# EUROPEAN PATENT APPLICATION

(11) **EP 0 615 752 A1**
(43) Date of publication of application: **21.09.1994**
(21) Application number: 94301654.3
(22) Date of filing: 09.03.1994
(51) Int. Cl.: A61K 31/20

(54) **Treatment of viral infections**

(30) Priority: 09.03.1993 GB 9304746
(71) Applicant: SCOTIA HOLDINGS PLC, Guildford Surrey GU1 1BA (GB)
(72) Inventor: Horrobin, David Frederick, c/o Scotia Pharm. Ltd., Guildford GU1 1BA, Surrey (GB); Winther, Michael David, c/o Efamol Res. Inc., Kentville, Nova Scotia B4N 4H8 (CA)
(74) Representative: Sturt, Clifford Mark

(57) **Abstract**

A method of treating viral infections, in particular retroviral infections, especially those with the HIV group of viruses, by destroying virus-infected cells in the body through administration of unsaturated fatty acids, particularly as lithium salts.

## Description

The present invention relates to treatment of viral infections, particularly retroviral infections, and more particularly to treatment of HIV infections and AIDS.

Viral infections are common and some, such as HIV infections are serious and increasing. In spite of much research, existing methods of treating viral infections are relatively limited, seeking to interfere with viral replication and transmission, and new techniques are urgently required. We have sought ways of treating viral infections which aim to destroy the cells which have been infected by the virus and thus to destroy the sites at which the viruses multiply. This new approach to treating viral infections is particularly appropriate when only a limited proportion of the normal cells in any class within the body is infected with the virus. This is true of HIV infections where only a small proportion of immune system cells (lymphocytes) is infected with the virus at any one time.

There is substantial evidence, presented for example in EP-A- 0,524,796 by the present applicants, that patients infected with HIV have reduced levels of certain essential fatty acid (EFAs). Treatment of patients having HIV infections is proposed therein using the EFA'S alone or in combination, though without any disclosed effect of selective destruction of virus infected cells. There are twelve naturally occurring EFAs, which are the n-6 and n-3 EFAs as shown in Figure 1. In EP-A-0,524,796, particular attention is drawn to GLA, DGLA, AA, EPA and DHA. Besides the above EFAs other unsaturated fatty acids such as parinaric and columbinic acid also have therapeutic effects.

The applicants have also previously drawn attention to the advantages of using the lithium salts when EFAs are being administered intravenously, for example in published European Application Nos. 0,585,057 and 0,585,058. The lithium salts are relatively water soluble and so can be added to ordinary aqueous intravenous (IV) solutions making their administration simple. Moreover, because the lithium ion is foreign to the body, at least in substantial amounts, it can be used to monitor the rates of infusion easily and quickly.

We have now found that unsaturated fatty acids and in particular their lithium salts have a unique effect on virally-infected lymphocytes. HIV infected lymphocytes are killed at physiologically achievable concentrations, whereas uninfected lymphocytes are not. This offers the possibility of eliminating virally infected cells from the body, while leaving normal cells unharmed, thus actually curing the viral infection as opposed to merely slowing down the rate of viral multiplication. The experiments have been performed with GLA, arachidonate, and eicosapentaenoate, but it is believed that at least the twelve n-6 and n-3 EFAs are likely to have similar effects, and also other unsaturated fatty acids such as parinaric acid and columbinic acid, and indeed unsaturated fatty acids with two or more unsaturations, generally. However, GLA, its immediate metabolite DGLA, EPA, and DHA are the preferred agents because of their safety when administered to normal tissue.

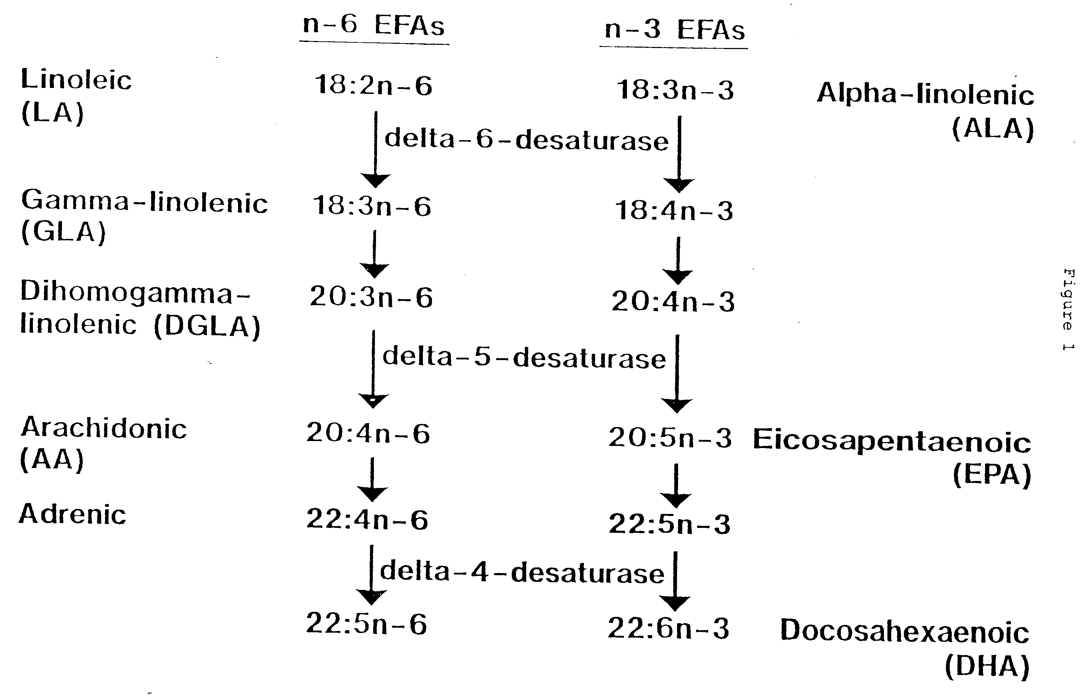

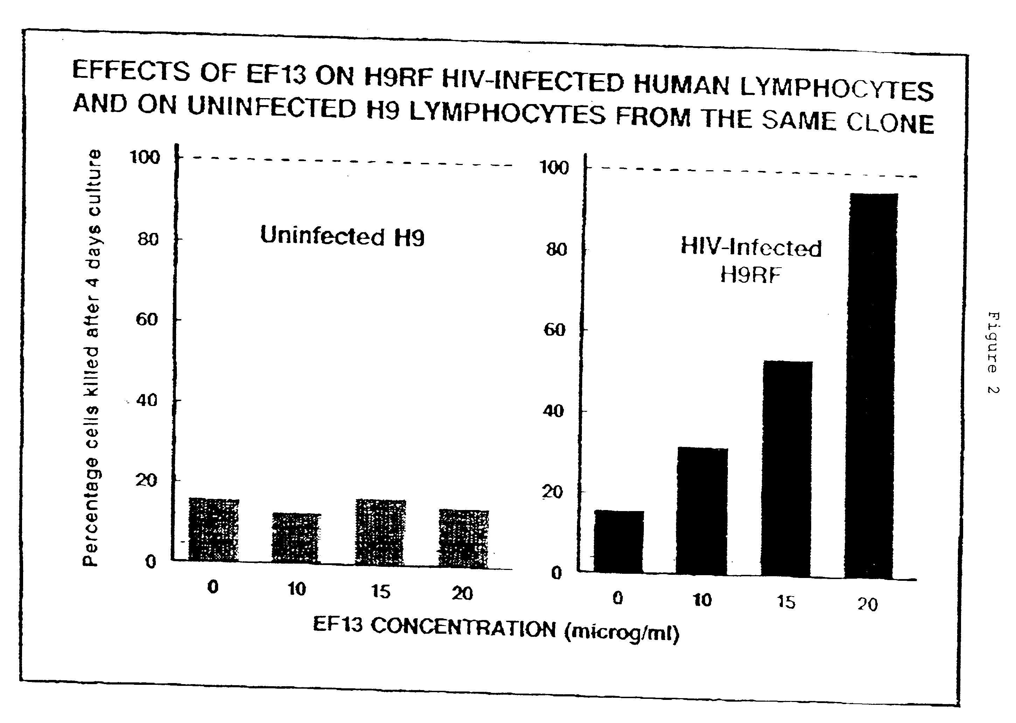

In a series of experiments, H9 normal human lymphocytes and H9RF cells chronically infected with a strain of HIV 1 were incubated with lithium-GLA at concentrations of 0, 10, 15, and 20 µg/ml. The cells were incubated for 4 days and the percentage of dead cells counted on days 3 and 4 using the trypan blue exclusion method. The results are shown in Figure 2 and are summarised below:
1. In the absence of any lithium-GLA both cell lines showed 10-20% dead cells at the end of 4 days.
2. All concentrations of lithium-GLA produced a similar result with the uninfected H9 cells, ie. 10-20% dead cells at the end of day 4. Lithium-GLA therefore had no toxic effect on the uninfected cells.
3. The infected H9RF HIV-infected cells, in contrast, showed progressively increasing cell death as the concentration of lithium-GLA increased. At a concentration of 20 µg/ml at 4 days, over 90% of the H9RF cells were killed.

These experiments therefore demonstrated that it is possible to kill HIV infected cells, so eliminating the reservoirs of infection, at doses of Li-GLA which do not harm normal cells. This effect is likely to occur with other retrovirus infections and other virus infections in general.

At its broadest the invention thus relates to a method of treating AIDS and HIV infections, other retroviral infections, and viral infections in general, by destruction of the virus infected cells through administration of unsaturated fatty acids, particularly as lithium salts; also and correspondingly to the use of unsaturated fatty acids, particularly the EFA's, preferably as the lithium salts thereof, in the preparation of a medicament for the treatment of AIDS and HIV infections, or after retroviral infections and viral infections generally, by destruction of the virus infected cells.

The preferred route of administering the drugs is by the intravenous route, but other routes such as oral or other parenteral routes may be used if appropriate. The daily dose may be for example from 1mg to 100g/day, preferably 500mg to 30g/day and very preferably 1g to 15g/day.

It is valuable to be able to maintain maximum safe plasma levels of unsaturated fatty acid levels by the methods of the published European Application Nos. 0,585,057 and 0,585,058, namely:
(a) Methods of administering fatty acid(s) intravenously, whereby the fatty acid(s) or a proportion thereof are administered in the form of lithium salts, and whereby the plasma lithium levels are regularly monitored to avoid haemolysis, preferably keeping the lithium level no higher than 0.7 millimolar in the long term and preferably also no higher than 0.4 - 0.5 millimolar in the first 2448 hours or, where only a proportion of the fatty acids are administered as lithium salts, a corresponding proportion of said levels.
(b) Methods of administering fatty acids intravenously whereby heparin in a dose of 1,000 - 20,000 IU, preferably 3,000 - 10,000 IU or equivalent anticoagulant dose of heparin-like protein or peptide is administered subcutaneously or intravenously prior to infusion of the fatty acid or where 12 - 100 IU, preferably 10 to 20 IU/ml heparin, or equivalent anticoagulant dose of heparin-like protein or peptide is added to the infusion fluid carrying the fatty acid, or where both pre-treatment of the patient with heparin and addition of heparin to the infusion fluid are combined.

The invention is further illustrated by the following examples.

### Examples

The following are used in treatment of AIDS or HIV or other retrovirus infections or virus infections generally, for the destruction of affected cells in the body:
1. Vials of solution for intraveneous administration containing lithium-GLA or lithium-DGLA in amounts of 1g to 5g, prepared as 10% or 20% solutions in 10% or 20% ethanol, one or two vials administered daily by slow intravenous infusions in isotonic saline.
2. Vials as in 1 but containing sodium salts of GLA or DGLA or any other appropriate soluble derivative of GLA or DGLA.
3. Vials as in 1 or 2 but containing like derivatives of any other EFA or other appropriate unsaturated fatty acid, either alone or in combination of two or more such acids.
4. Capsules, tablets, pastilles, drogues, or any other such appropriate oral dosage forms for EFAs or other appropriate unsaturated fatty acids as above as such or as derivatives, for administration of 0.5g to 1.5g of the fatty acid per dosage unit, 3g to 15g total per day.
5. Oils, foams, whips, mousses, granules, powders, or any other such appropriate oral dosage forms for EFAs or other appropriate unsaturated fatty acids as above as such or as derivatives, for administration of 0.5g to 1.5g of the fatty acid per dosage unit, 3g to 15g total per day.
6. Ointments, creams, lotions, oils, shampoos or any other appropriate topical dosage forms containing appropriate concentrations of any of the EFAs or other appropriate unsaturated fatty acids or derivatives thereof for topical administration, concentrations being for example 0.1 to 10% by weight calculated as the fatty acid for convenient administration of for example 3g to 15g of fatty acid daily.

## Claims

1. A method of treating AIDS and HIV infections by destroying virus-infected cells by the administration to a person suffering from such infection of an effective amount of one or more unsaturated fatty acids, as such or as derivatives particularly as lithium salts, especially the twelve n-6 and n-3 EFAs and more especially GLA or DGLA or EPA or DHA.

2. A method according to claim 1 in which administration is of 1mg to 100g of the or each fatty acid daily, desirably 500mg to 30g and more desirably 1g to 15g, preferably by the intravenous route.

3. A method of treating retroviral infections other than HIV infections by the method set out in claim 1.

4. A method of treating any viral infection by the method set out in claim 1.

5. Use of one or more unsaturated fatty acids, particularly as the lithium salts thereof, in the preparation of a medicament for the treatment of AIDS and HIV infections, or other retroviral infections, or viral infections generally, by destruction of virus infected cells in the body.

6. Use as in claim 5 of the twelve n-6 and n-3 EFA's and more especially GLA or DGLA or EPA or DHA.
